(19) Europäisches Patentamt
European Patent Office
Office européen des brevets

(11) **EP 3 718 796 B1**

(12) **EUROPEAN PATENT SPECIFICATION**

(45) Date of publication and mention
of the grant of the patent:
**23.11.2022 Bulletin 2022/47**

(21) Application number: **19167631.1**

(22) Date of filing: **05.04.2019**

(51) International Patent Classification (IPC):
***B60H 1/00*** *(2006.01)*

(52) Cooperative Patent Classification (CPC):
**B60H 1/00742;** B60H 1/00807

(54) **EVALUATION SYSTEM FOR THE CONDITION EVALUATION OF A PASSENGER**

AUSWERTESYSTEM FÜR DIE ZUSTANDSBEWERTUNG EINES PASSAGIERS

SYSTÈME D'ÉVALUATION DE L'ÉTAT D'UN PASSAGER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**07.10.2020 Bulletin 2020/41**

(73) Proprietor: **Ford Global Technologies, LLC
Dearborn, MI 48126 (US)**

(72) Inventors:
• **Dienhart, Bernd
50859 Köln (DE)**
• **Scheer, Volker
52159 Roetgen (DE)**
• **Maranville, Clay Wesley
Ypsilanti, MI 48197 (US)**
• **Elson, John Craig
Farmington Hills, MI 48334 (US)**
• **Schuermanns, Klaus
50739 Köln (DE)**
• **Becker, Christian
53604 Bad Honnef (DE)**
• **Krolewski, Sandra
41469 Neuss (DE)**
• **Massonet, Christoph
52064 Aachen (DE)**
• **Backes, Damian
52070 Aachen (DE)**

• **Hirn, Thomas
52062 Aachen (DE)**
• **Wesseling, Mark
52062 Aachen (DE)**
• **Rewitz, Kai
52066 Aachen (DE)**
• **Seiwert, Paul
52072 Aachen (DE)**

(74) Representative: **Dörfler, Thomas
Ford-Werke GmbH
Abt. NH/364
Henry-Ford-Strasse 1
50735 Köln (DE)**

(56) References cited:
EP-A1- 3 284 622     WO-A1-2014/085302
FR-A1- 3 065 915     US-A- 5 187 943
US-A- 5 674 007       US-A1- 2016 089 955

• **ANONYMOUS: "ISO 7730:2005 Ergonomics of the
thermal environment -- Analytical determination
and interpretation of thermal comfort using
calculation of the PMV and PPD indices and local
thermal comfort criteria", ISO STANDARD, ISO,
CH , 15 November 2005 (2005-11-15), pages 1-52,
XP009516092, Retrieved from the Internet:
URL:https://www.iso.org/standard/39155.htm l
[retrieved on 2019-09-17]**

**Description**

The present invention refers to an evaluation system for the condition evaluation of a vehicle passenger, and to a method for evaluating the condition of a vehicle passenger.

The heating ventilation and air conditioning system (HVAC) of a vehicle has to fulfill certain requirements, like window defrosting, demisting as well as heating and cooling of the passenger cabin. In addition, todays intelligent electronic automatic temperature control systems (EATC) provide a plurality of features, such as cabin air temperature and humidity control, air flow and air distribution management, multizone climate control, sun load compensation, automatic fog detection and/or control of the system towards optimized energy efficiency.

Passenger comfort is currently assumed at a certain air temperature and associated airflow inside the vehicle cabin, which can be selected and interactively changed by the passenger through the EATC control head. In an auto-mode operation, the passenger may select an interior temperature setting, which is expected to generate a comfortable climate within a certain period of time. The vehicle cabin climate is expected to stay comfortable during the respective trip. Changing parameters during the trip, such as vehicle speed, ambient conditions, sun load, tunnels, or the like must be compensated by the EATC, preferably without any impact to the passenger comfort.

To reach a comfortable climate inside the passenger cabin under various conditions, high integration effort of the respective sensors and a high number of evaluation drives are required under different ambient conditions and through simulation of expected customer behavior. Such calibration procedure for current EATC systems regarding air flow, air distribution and air temperature control inside the vehicle needs to be done for each vehicle line and each vehicle cabin separately. This requires high efforts and costs.

Apart from the above, the very indirect way of calibration may still cause passenger discomfort under certain conditions, particularly in case of unsuitable calibration or in case of conditions not considered for calibration.

It is already known to install infrared sensors in the cabin of vehicle, in order to improve the climate control functionality of HVAC systems. However, such infrared sensors are typically cost intensive. This particularly holds true for infrared sensors with a high resolution. Furthermore, the correlation between infrared measurements and discharge temperatures of a HVAC system may still lead to passenger discomfort under certain conditions.

It is also known from US 5674007A to evaluate the condition of a vehicle passenger based on multiple parameters including air temperature, a passenger clothing state, a passenger metabolic rate and a passenger thermal body state.

Against this background it has been the objective of the present invention to provide an evaluation system as well as a method for evaluating the condition of a vehicle passenger, which allow to reduce the risk of passenger discomfort and/or reduce the risk of unsuitable climate conditions within a vehicle.

The above mentioned objective has been solved by the features of claims 1 and 14. Preferred embodiments are laid down in the dependent claims, and will be discussed in the following.

An evaluation system according to the present invention is configured for the condition evaluation of a vehicle passenger. Such a condition evaluation may, for example, refer to a comfort condition and/or an emergency condition of a vehicle passenger, but is not limited thereto.

According to the present invention, the evaluation system comprises a parameter evaluation device being configured to evaluate a plurality of parameters of a set of vehicle interior and vehicle passenger parameters, the set of parameters including:

- a vehicle cabin air temperature parameter,
- a vehicle cabin surface temperature parameter,
- a passenger thermal body state parameter,
- a passenger metabolic rate parameter,
- a passenger clothing level parameter,
- a passenger respiratory rate parameter, and
- a passenger mood state parameter.

The evaluation system according to the present invention furthermore comprises a condition value evaluation device being configured to evaluate a passenger condition value based on the parameters evaluated by the parameter evaluation unit. The condition value evaluation device is configured to apply a weighting factor for a parameter evaluated by the parameter evaluation device. Said weighting factor is within one of the ranges of:

$$-10 < a < 10,$$

$$-10 < b < 10,$$

$$0 < c < 8,$$

$$0 < d < 8,$$

$$0 < e < 5,$$

$$-1 < f < 0,$$

and

$$0.2 < g < 3;$$

where:

a = vehicle cabin air temperature parameter weighting factor,
b = vehicle cabin surface temperature parameter weighting factor,
c = passenger thermal body state parameter weighting factor,
d = passenger metabolic rate parameter weighting factor,
e = passenger clothing level parameter weighting factor,
f = passenger respiratory rate parameter weighting factor, and
g = passenger mood state parameter weighting factor.

The evaluation system according to the invention is characterized in that the condition value evaluation device is configured to evaluate a passenger condition value based on the function:

$$CV = a \cdot (T_{cabin,\,air} - T_{cabin,air,ref}) + b \cdot (T_{cabin,\,rad} - T_{cabin,rad,ref}) + c \cdot TBS +$$
$$d \cdot (MR - MR_{ref}) + e \cdot (CL - CL_{ref}) + f \cdot (RR - RR_{ref}) + g \cdot MS$$

where:

CV = passenger condition value,
$T_{cabin,air,ref}$ = reference vehicle cabin air temperature parameter,
$T_{cabin,rad,ref}$ = reference vehicle cabin surface temperature parameter,
$MR_{ref}$ = passenger metabolic rate reference value,
$CL_{ref}$ = passenger clothing level reference value,
$RR_{ref}$ = passenger respiratory rate reference value.

With an evaluation system according to present invention, a passenger condition value representing detailed condition information may be evaluated. The system allows a flexible and highly individualized condition evaluation, particularly in view of a high number of relevant parameters, while at the same time ensuring their suitable and/or precise consideration for different vehicle functionalities, such as the functionality of an HVAC system, an interior lighting system, an infotainment system, an emergency system and/or other systems of a vehicle. The consideration of a rather high number of detected and/or evaluated parameters widens the data base on a passengers state and/or a vehicle interior state, and subsequently allows to improve the respective functionalities of the vehicle, particularly comfort related functionalities.

In the present context, the term "passenger" may refer to occupants in general, including, for example, a driver, a co-driver, and/or a rear-passenger.

According to a preferred embodiment of the present invention, at least one weighting factor for a parameter evaluated by the by the parameter evaluation device may be within the range of

$$-8 < a < 8,$$

$$-7 < a < 7,$$

$$-8 < b < 8,$$

$$-7 < b < 7,$$

$$0 < c < 6,$$

$$0 < c < 5,$$

$$0.2 < c < 5,$$

$$0 < d < 6,$$

$$0 < d < 5,$$

$$1 < d < 5,$$

$$0 < e < 4,$$

$$0 < e < 3,$$

$$0.6 < e < 3,$$

$$-0.5 < f < 0,$$

$$-0.3 < f < 0,$$

$$0.3 < g < 2$$

and/or

$$0.5 < g < 1;$$

where:

a = vehicle cabin air temperature parameter weighting factor,
b = vehicle cabin surface temperature parameter weighting factor,
c = passenger thermal body state parameter weighting factor,

d = passenger metabolic rate parameter weighting factor,
e = passenger clothing level parameter weighting factor,
f = passenger respiratory rate parameter weighting factor, and
g = passenger mood state parameter weighting factor.

According to the invention, the condition value evaluation device is configured to evaluate a passenger comfort condition value, for example a thermal sensation vote. The evaluation system may particularly be suitable for providing a condition value as an input factor for the HVAC system of a vehicle. The passenger comfort may thereby suitably improved. Individual comfort assessment for each passenger, represented by a passenger comfort condition value, for example in the form of a thermal sensation vote, may be used for individual control of discharge temperature, air distribution and/or air velocity of an HVAC system, and therewith improve the comfort of the respective vehicle passenger.

According to a yet preferred embodiment, the condition value evaluation device may be configured to evaluate a passenger emergency condition value. Such an emergency condition value, for example, representing an health emergency such as a heart attack or cardiac arrest, may be used as an input factor for a vehicle emergency system. The latter may for example be configured for the initiation of an automated emergency call. Passenger safety may therewith be improved.

According to a yet preferred embodiment, the condition value evaluation device may be configured to provide the evaluated condition value to an electronic automatic temperature control system (EATC) and/or to an infotainment and/or communication system of a vehicle. An EATC system may use the condition value for automated temperature control, and a communication system may use an emergency related condition value for a possible emergency related operation, such as an emergency call. The functionality may therewith be further enhanced.

According to a yet preferred embodiment, the condition value evaluation device may be configured to evaluate a passenger condition value based on a plurality of remaining parameters evaluated by the parameter evaluation unit in case of any missing parameter and/or any parameter evaluation malfunction. The operational safety of the evaluation system may thereby be improved, particularly avoiding a total malfunction due to the missing of single parameters and/or single parameter evaluation malfunctions.

According to a yet preferred embodiment, the condition value evaluation device may be configured to apply a predefined substitute parameter value for any missing parameter and/or in case of any parameter evaluation malfunction. That is, in case an intended parameter evaluation is not successful, a predefined substitute parameter value may be applied, in order to avoid significant falsifications of the final condition value. Such predefined substitute parameter value may be saved permanently within the parameter evaluation device and/or within the condition value evaluation device and/or adapted automatically and/or manually.

According to a yet preferred embodiment, the condition value evaluation device may be configured to apply a weighting factor for each substitute parameter value, said weighting factor corresponding to the weighting factor of the substituted parameter. That is, in case of application of a substitute parameter value, the same weighting factor, which would have been applied for the corresponding parameter, is applied for the substitute parameter value. The risk of falsifications of the final condition value may therewith be further reduced. According to a yet preferred embodiment, the condition value evaluation device may be configured to evaluate a passenger condition value based on at least one predefined parameter reference value, the reference value being within one of the ranges of:

$$16\,°C < T_{cabin,air,ref} < 26\,°C,$$

$$16\,°C < T_{cabin,rad,ref} < 26\,°C,$$

$$1 < MR_{ref} < 1.6,$$

$$0.8 < CL_{ref} < 1.5,$$

and/or

$$12 < RR_{ref} < 15,$$

where

$T_{cabin,air,ref}$ = reference vehicle cabin air temperature,
$T_{cabin,rad,ref}$ = reference vehicle cabin surface temperature,
$MR_{ref}$ = passenger metabolic rate reference value,
$CL_{ref}$ = passenger clothing level reference value, and
$RR_{ref}$ = passenger respiratory rate reference value.

By applying such parameter reference values, the evaluation precision may be further improved. The above mentioned parameter reference values may be saved permanently within the parameter evaluation device and/or within the condition value evaluation device. The parameter reference values may be adaptable automatically and/or manually and/or be permanently fixed to a predefined value, particularly unchangeable by a vehicle passenger. According to a yet preferred embodiment, the parameter evaluation device may be configured to evaluate a vehicle cabin air temperature parameter based on at least one temperature measurement of a vehicle interior surface, preferably of a surface with low thermal inertia. Further preferred, the parameter evaluation device may be configured to evaluate a vehicle cabin air temperature parameter based on at least one temperature measurement of a fabric surface and/or a headrest surface and/or a backrest surface and/or a roof lining surface. Such temperature measurement may be conducted with limited effort and high operational safety.

According to a further preferred embodiment, the parameter evaluation device may be configured to evaluate a vehicle cabin air temperature parameter based on a plurality of temperature measurements of different vehicle interior surfaces. The evaluation accuracy may thereby be improved.

In a further preferred embodiment, the parameter evaluation device may be configured to evaluate a vehicle cabin air temperature parameter based on the function:

$$T_{cabin,air} = \sum_{i=1}^{..} w_i \cdot T_{surf,i}$$

with

$$0 < w_i < 1$$

and

$$\sum w_i = 1$$

and where

$T_{cabin,air}$ = vehicle cabin air temperature parameter,
$T_{surf,i}$ = measured vehicle cabin surface temperature, and
$w_i$ = weighting factor for a vehicle interior surface.

Evaluations of a vehicle cabin air temperature parameter based on this function may be conducted with only little effort, while at the same time providing sufficiently accurate results.

According to a further embodiment, the parameter evaluation device may be configured to evaluate a vehicle cabin surface temperature parameter based on at least one temperature measurement of a vehicle interior surface, preferably based on at least one temperature measurement of vehicle cabin surface .

More preferably, the parameter evaluation device may be configured to evaluate a vehicle cabin surface temperature parameter based on at least one temperature measurement of a dashboard, a ceiling portion, a window portion, and/or a door portion. Such surface portions may have a particularly high thermal inertia, and may therefore provide precise information on the surface temperatures within a vehicle cabin and/or temperature radiation from such surfaces.

Furthermore, the emissivity coefficient of the respective surface portion may be previously determined and/or suitably predefined and/or permanently saved within the parameter evaluation device and/or within the condition value evaluation device. This allows to accurately assess the respective surface temperature and/or the respective surface temperature parameter. According to a further preferred embodiment, the vehicle cabin surface temperature parameter weighting factor may be based on at least one or a plurality of emissivity values of respective surfaces. The evaluation accuracy may thereby further improved.

According to a further embodiment, the parameter evaluation device may be configured to evaluate a vehicle cabin surface temperature radiation parameter. That is, any evaluated vehicle cabin surface temperature parameter may be a vehicle cabin surface temperature radiation parameter. Accordingly, the evaluated parameter may represent any possible temperature radiation from cabin surfaces.

According to a further embodiment, the parameter evaluation device may be configured to evaluate a vehicle cabin surface temperature parameter based on a plurality of temperature measurements of different vehicle interior surfaces. The evaluation accuracy may be further improved and the risk of falsifications by single measurements reduced.

Particularly preferred, the parameter evaluation device may be configured to evaluate a vehicle cabin surface temperature parameter based on the function:

$$T_{cabin,rad} = \sum_{i=1}^{..} w_i \cdot T_{surf,i};$$

with

$$0 < w_i < 1$$

and

$$\sum w_i = 1$$

and where

$T_{cabin,rad}$ = vehicle cabin surface temperature parameter,
$T_{surf,i}$ = measured vehicle cabin surface temperature, and
$w_i$ = weighting factor for a vehicle interior surface.

Evaluations of a surface temperature parameter based on this functions may be conducted with only little effort, while at the same time providing sufficiently accurate results.

According to a yet further preferred embodiment, the parameter evaluation device may be configured to evaluate a thermal body state parameter, a metabolic rate parameter, a clothing level parameter and/or a respiratory rate parameter based on at least one computer vision algorithm and/or based on at least one measurement of a passenger's head and/or other body parts. Preferably measurements of an arm, a chest and/or a hand of a vehicle passenger may be taken as a basis for the parameter evaluation by the parameter evaluation device. Thereby, detailed information on the condition of a passenger may be acquired with only limited effort. The parameters respectively evaluated may serve for the evaluation of a suitable passenger condition value containing a high level of information.

According to a yet further preferred embodiment, the parameter evaluation device may be configured to evaluate a thermal body state parameter based on at least one measured skin temperature in a region of interest, preferably of an unclothed body part and/or of a facial, arm and/or hand portion of a vehicle passenger and/or of a body part exposed to solar radiation. This allows a high degree of evaluation accuracy and evaluation safety, while at the same time requiring only limited evaluation effort.

Further preferred, the parameter evaluation device may be configured to evaluate a local and/or a global thermal body state parameter. A local thermal body state parameter may provide detailed information on single body portions of a vehicle passenger and a global thermal body state may provide detailed information on the overall condition of a vehicle passenger. In case both types of thermal body state parameters may be evaluated, a high degree of evaluation flexibility may be achieved.

According to a further preferred embodiment, the parameter evaluation device may be configured to evaluate a thermal body state parameter based on a weighted average of skin temperature measurements as well as a temporal gradient of the skin temperature, preferably a temporal gradient of the skin temperature in the facial area of the passenger. Thereby, a global and/or a local thermal body state parameter may be evaluated with only limited effort and at the same time with high accuracy.

Furthermore, the parameter evaluation device may preferably be configured to evaluate a thermal body state parameter based on the function:

$$\text{TBS} = a_i \cdot \left( T_{skin,\,i} - T_{skin,ref} \right) + b_i \cdot \frac{dT_{skin,\,i}}{dt}$$

with

$$0.2 < a_i < 2$$

$$2 < b_i < 40$$

$$29\,°C < T_{skin,ref} < 35°C,$$

and

$$T_{skin,i} \text{ in } °C;$$

where

TBS = passenger thermal body state parameter,
$T_{skin,i}$ = measured passenger skin temperature,
Tskin,ref = passenger skin reference temperature.

Evaluations of the passenger thermal body state parameter based on this functions may be conducted with only little effort, while at the same time providing sufficiently accurate results.
According to a further preferred embodiment, the parameter evaluation device may be configured to evaluate a clothing level parameter based on at least one temperature measurement of a local clothing surface in the region of interest of a clothed body part, preferably of a chest and/or arm portion. This allows a simple and effective evaluation of a clothing level parameter.
Preferably, the parameter evaluation device may be configured to evaluate a clothing level parameter based on the function:

$$CL = \left( \sum_{i=0}^{n} z_i T_{Cabin,air}^{i} \right) \left( 1 + T_{body\_surf_j} \right) + \sum_{i=0}^{m} c_i T_{body\_surf}^{i}$$

where:

CL = passenger clothing level parameter,
$T_{cabin,air}$ = vehicle cabin air temperature parameter,
$T_{body\_surf,i}$ = measured passenger body surface temperature,
and
$z_i$ = coefficients.

The coefficients $z_i$ may be derived from empirical correlations and/or predefined on the basis of empirical correlations.
Preferably, the parameter evaluation device may also be configured to evaluate a clothing level parameter based on the function:

$$CL = ((z_1 \cdot T_{cabin,\,air})^2 + z_2 \cdot T_{cabin,\,air} + z_3) \cdot T_{body\_surf,\,i} + z_4 \cdot T_{cabin,\,air}^2 + z_5 \cdot T_{cabin,\,air} + z_6$$

where:

CL = passenger clothing level parameter,
$T_{cabin,air}$ = vehicle cabin air temperature parameter,
$T_{body\_surf,i}$ = measured passenger body surface temperature, and
$z_1$ to $z_6$ = coefficients.

The coefficients $z_1$ to $z_6$ may be derived from empirical correlations and/or predefined on the basis of empirical correlations.

The evaluation of the clothing level parameter based on the above function may be conducted with only little effort, while at the same time providing sufficiently accurate results.

According to a further preferred embodiment, the parameter evaluation device may be configured to evaluate a respiratory rate parameter based on measured temperature fluctuations in the sub-nasal area of the face of a vehicle passenger and/or based on temperature fluctuations caused by alternating airflow through the passenger nose caused by respiratory activity. This allows to reliably evaluate a respiratory rate parameter with only limited effort.

Further preferred, the parameter evaluation device may be configured to evaluate a respiratory rate parameter using a band pass filter and/or a Fast-Fourier-Transformation. The evaluation accuracy may thereby be further improved and evaluation effort kept limited.

According to a further preferred embodiment, the parameter evaluation device may be configured to evaluate a metabolic rate parameter based on an evaluated heart beat rate of a vehicle passenger. The heart beat rate may provide a reliable evaluation basis for the metabolic rate parameter.

Furthermore, the parameter evaluation device may be configured to evaluate a heart beat rate based on measurements and/or monitoring results of a vehicle passenger forehead. A passenger forehead may provide reliable information on the heart beat rate of the respective passenger.

Preferably, the parameter evaluation device may be configured to evaluate a heart beat rate by measurement of temperature fluctuations caused by the variations in blood flow through the hypodermic blood vessels of a passenger forehead. Such temperature fluctuations may be suitably measured with a framerate of at least 2 fps, more preferably at least 3 fps. The evaluation device may be configured for such framerate measurements.

The parameter evaluation device may also be configured to evaluate a heart beat rate by measurement of color variations of a vehicle passenger forehead skin, preferably magnified by Eulerian Video Magnification algorithms. Such measurements may be conducted with limited effort and high accuracy.

Moreover, the parameter evaluation device may be configured to evaluate a metabolic rate parameter using a band pass filter and/or a Fast-Fourier-Transformation. The evaluation accuracy may thereby be further improved and evaluation effort kept limited.

According to a further preferred embodiment, the parameter evaluation device may be configured to evaluate a mood state parameter based on optical tracking of facial landmarks, preferably eyes, eyebrows, a nose and/or a mouth of a vehicle passenger. Such facial landmarks may provide a reliably information basis for a mood state parameter. For example, an angry or friendly look of a passenger may reliably be evaluated based on optical tracking of such facial landmarks.

Preferably, the parameter evaluation device may be configured to evaluate a mood state parameter based on a transformation of relative geometric positions of tracked facial landmarks. The evaluation accuracy may thereby be further improved.

The parameter evaluation device and/or the condition value evaluation device may be provided in the form of a single control device or multiple control devices. Such a control device may comprise a CPU and a data storage device. The data storage device may inter alia comprise suitable programming code, particularly for providing the required functionality of the controller. The parameter evaluation device may comprise a plurality of different parameter evaluation units. Said units may be provided by different portions of a programming code or software portions. It is also possible that the different parameter evaluation units are provided by physically distinct controllers.

According to a yet further preferred embodiment, the evaluation system may further comprise a condition sensor device, preferably configured for optical and/or temperature measurement. The condition sensor device may comprise at least one optical sensor unit and/or at least one surface temperature sensor unit. Different sensor units may provide different sensing functionalities. By providing an optical sensor unit, certain body portions or cabin surface portions may be detected with limited effort. For these detected portions temperature measurements with surface temperature sensor unit may be conducted. The pixel density of a surface temperature sensor unit may thereby kept small, while at the same time ensuring a sufficient measuring accuracy. Particularly, the combination of the two different units allows the use of a cost-effective temperature sensor unit. The two units may be integrated into one single sensor device or arranged as two separate units. The surface temperature sensor unit may be a longwave infrared sensor unit for surface temperature measurements, such as a pyrometer or infrared temperature sensor. Furthermore, the condition sensor device may be configured to provide sensor signals to the parameter evaluation device.

According to the present invention, the evaluation system, comprises a parameter evaluation device being configured to evaluate a plurality of parameters of a set of vehicle interior and vehicle passenger parameters, the set of parameters including:

- environmental parameters of a vehicle interior,
- vehicle passenger physiological parameters, and
- at least one vehicle passenger psychological parameter.

According to the present invention, environmental parameters of a vehicle interior are a vehicle cabin air temperature parameter and a vehicle cabin surface temperature parameter.

According to the present invention, vehicle passenger physiological parameters are a passenger thermal body state parameter, a passenger metabolic rate parameter, a passenger clothing level parameter, and a passenger respiratory rate parameter

According to the present invention, a vehicle passenger psychological parameters is a passenger mood state parameter.

The evaluation system according to the invention further comprises a condition value evaluation device being configured to evaluate a passenger condition value based on at least the parameters evaluated by the parameter evaluation unit.

According to a further aspect of the present invention, the above described evaluation system may comprise a condition sensor device, wherein the condition sensor device may comprise at least one optical sensor unit for sensing wavelength in the visible spectrum, and at least one temperature sensor unit for sensing wavelengths in the long-wave infrared spectrum, wherein the pixel density of the optical sensor unit is at least 4 times higher, preferably at least 5 times higher, than the pixel density of the temperature sensor unit. The above mentioned difference of pixel densities allows to use a particularly cost-effective temperature sensor unit, while at the same time ensuring sufficiently high resolutions by additionally using optical sensor unit with a significant higher pixel density. Optical sensor units may still be provided at low costs even in case of relatively high pixel densities.

In the present context, the term pixel density may be understood as the relation between the field of view and the number of pixels.

Furthermore, the long-wave infrared spectrum comprises wavelengths between 7 $\mu$m and 15 $\mu$m, particularly between 8 $\mu$m and 14 $\mu$m. Accordingly, long-wave infrared radiation may refer to a radiated heat, preferably - but not limited to - a wavelength of 8 $\mu$m to 14 $\mu$m.

According to a further aspect of the present invention the minimum pixel density of the temperature sensor unit may be defined by the function:

$$\frac{pixel}{deg} \bigg| \min = d \, \frac{\pi}{180°} \frac{\text{HP}}{\text{WF}}$$

where

    d = distance between the temperature sensor and a passenger face,
    HP = minimum number of horizontal pixels for a face resolution, and
    WF = average width of a passenger face.

Alternatively, the minimum pixel density of the temperature sensor unit may be defined by the function:

$$\frac{pixel}{deg} \bigg| \min = d \, \frac{\pi}{180°} \frac{\text{VP}}{\text{HF}}$$

where

d = distance between the temperature sensor and a passenger face,
VP = minimum number of vertical pixels for a face resolution, and
HF = average height of a passenger face.

Furthermore, in the present context a preferable range of pixel per deg may be between 0.05 and 10 in horizontal direction.

Furthermore, in the present context a preferable range of pixel per deg may be between 0.05 and 10 in vertical direction.

A further aspect of the present invention refers to a method for evaluating the condition of a vehicle passenger with an above described evaluation system, the method comprising the steps of:

- evaluating a plurality of parameters of a set of vehicle interior and vehicle passenger parameters, the set of parameters including:

  - a vehicle cabin air temperature parameter,
  - a vehicle cabin surface temperature parameter,
  - a passenger thermal body state parameter,
  - a passenger metabolic rate parameter,
  - a passenger clothing level parameter,
  - a passenger respiratory rate parameter, and
  - a passenger mood state parameter; and
- evaluating a passenger condition value based on at least the plurality of evaluated parameters;
- wherein the evaluation of the condition value is conducted by applying a weighting factor for an evaluated parameter; the weighting factor being within one of the ranges of:

$$-10 < a < 10,$$

$$-10 < b < 10,$$

$$0 < c < 8,$$

$$0 < d < 8,$$

$$0 < e < 5,$$

$$-1 < f < 0,$$

and

$$0.2 < g < 3;$$

where:

a = vehicle cabin air temperature parameter weighting factor,
b = vehicle cabin surface temperature parameter weighting factor,
c = passenger thermal body state parameter weighting factor,
d = passenger metabolic rate parameter weighting factor,
e = passenger clothing level parameter weighting factor,
f = passenger respiratory rate parameter weighting factor, and
g = passenger mood state parameter weighting factor.

The method according to the invention is characterized in that a passenger condition value (CV) is evaluated based on the function:

$$CV = a \cdot (T_{cabin,\,air} - T_{cabin,air,ref}) + b \cdot (T_{cabin,\,rad} - T_{cabin,rad,ref}) + c \cdot TBS +$$
$$d \cdot (MR - MR_{ref}) + e \cdot (CL - CL_{ref}) + f \cdot (RR - RR_{ref}) + g \cdot MS$$

where:

CV = passenger condition value,
$T_{cabin,air,ref}$ = reference vehicle cabin air temperature parameter,
$T_{cabin,rad,ref}$ = reference vehicle cabin surface temperature parameter,
$MR_{ref}$ = passenger metabolic rate reference value,
$CL_{ref}$ = passenger clothing level reference value,
$RR_{ref}$ = passenger respiratory rate reference value.

According to a preferred embodiment, the evaluation of the condition value is conducted by applying at least one weighting factor within the range of:

$$-8 < a < 8,$$

$$-7 < a < 7,$$

$$-8 < b < 8,$$

$$-7 < b < 7,$$

$$0 < c < 6,$$

$$0 < c < 5,$$

$$0.2 < c < 5,$$

$$0 < d < 6,$$

$$0 < d < 5,$$

$$1 < d < 5,$$

$$0 < e < 4,$$

$$0 < e < 3,$$

$$0.6 < e < 3,$$

$$-0.5 < f < 0,$$

$$-0.3 < f < 0,$$

$$0.3 < g < 2$$

and/or

$$0.5 < g < 1;$$

where:

a = vehicle cabin air temperature parameter weighting factor,
b = vehicle cabin surface temperature parameter weighting factor,
c = passenger thermal body state parameter weighting factor,
d = passenger metabolic rate parameter weighting factor,
e = passenger clothing level parameter weighting factor,
f = passenger respiratory rate parameter weighting factor, and
g = passenger mood state parameter weighting factor.

The above explanations and details regarding the evaluation system apply also to the method for evaluating the condition of a vehicle passenger.

Other embodiments of the invention result from combinations of the features disclosed in the claims, the specification and the drawings.

Fig. 1     is a schematic diagram of an evaluation device according to an embodiment of the present invention,
Fig. 2     is a schematic diagram of a condition sensor device arranged within a passenger cabin of a vehicle,
Fig. 3     is a schematic diagram of a condition sensor device positioned at a distance from a passenger face,
Fig. 4a    shows a temperature measurement of a person with a face resolution of 30x20 pixels,
Fig. 4b    shows a temperature measurement of a person with a face resolution of 12x8 pixels,
Fig. 4c    shows a temperature measurement of a person with a face resolution of 6x4 pixels.

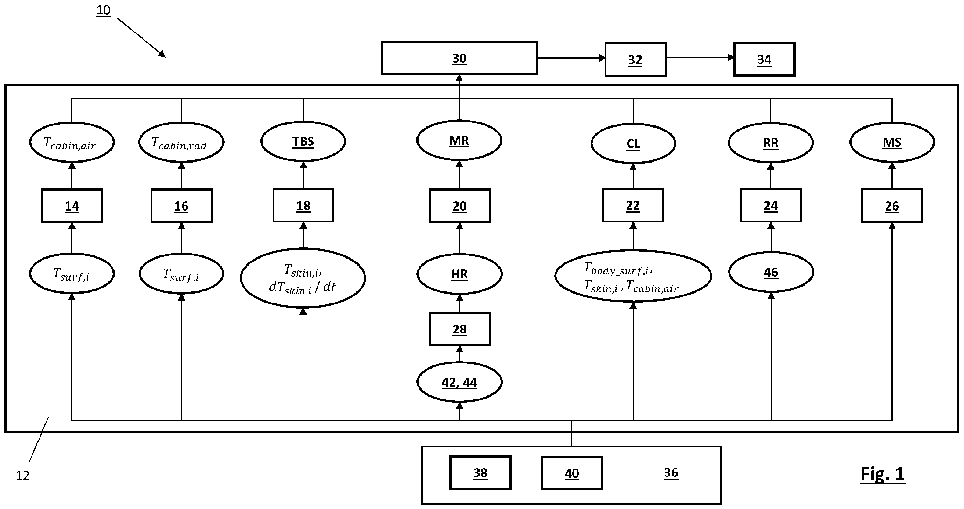

Fig. 1 is a schematic diagram of an evaluation device 10 according to an embodiment of the present invention. The evaluation device 10 shown in Fig. 1 is configured for the condition evaluation of a vehicle passenger. Such condition evaluation of a vehicle passenger may include the evaluation of a passenger comfort condition value, preferably a thermal sensation vote, and/or a passenger emergency condition value.

The evaluation device 10 shown in Fig. 1 comprises a parameter evaluation device 12 being configured to evaluate a plurality of parameters of a set of vehicle interior and vehicle passenger parameters. The set of parameters includes a vehicle cabin air temperature parameter, a vehicle cabin surface temperature parameter, a passenger thermal body state parameter, a passenger metabolic rate parameter, a passenger clothing level parameter, a passenger respiratory rate parameter, and a passenger mood state parameter. The set of parameters may also include passenger heart beat rate parameter.

For the evaluation of the above mentioned parameters, the parameter evaluation device 12 may comprise a vehicle cabin air temperature parameter evaluation unit 14, a vehicle cabin surface temperature parameter evaluation unit 16, a passenger thermal body state parameter evaluation unit 18, a passenger metabolic rate parameter evaluation unit 20, a passenger clothing level parameter evaluation unit 22, a passenger respiratory rate parameter evaluation unit 24, and/or a passenger mood state parameter evaluation unit 26. The parameter evaluation device 12 may also comprise a heart beat rate evaluation unit 28.

Furthermore, the evaluation device 10 according to the embodiment in Fig. 1 comprises a condition value evaluation device 30 being configured to evaluate a passenger condition value based on at least the parameters evaluated by the parameter evaluation unit 12. The condition value evaluation device 30 is configured to evaluate a passenger comfort condition value, preferably a thermal sensation vote, and/or a passenger emergency condition value.

The condition value evaluation device 30 is configured to apply a weighting factor for a parameter evaluated by the parameter evaluation device 12, the weighting factor being within one of the ranges of:

$$-10 < a < 10,$$

$$-10 < b < 10,$$

$$0 < c < 8,$$

$$0 < d < 8,$$

$$0 < e < 5,$$

$$-1 < f < 0,$$

and

$$0.2 < g < 3;$$

where:

a = vehicle cabin air temperature parameter weighting factor,
b = vehicle cabin surface temperature parameter weighting factor,
c = passenger thermal body state parameter weighting factor,
d = passenger metabolic rate parameter weighting factor,
e = passenger clothing level parameter weighting factor,
f = passenger respiratory rate parameter weighting factor, and
g = passenger mood state parameter weighting factor.

The consideration of the different parameters allows an holistic evaluation of a passenger condition. At the same time, the above weighting factors may ensure that the parameters are put in a suitable and/or realistic relation to each other, in order for the final condition value to be accurate and suitable for any further control operation.

Furthermore, the condition value evaluation device 30 may be configured to evaluate a passenger condition value CV based on at least one predefined parameter reference value, the reference value being within one of the ranges of:

$$16\ °C < T_{cabin,air,ref} < 26\ °C,$$

$$16\ °C < T_{cabin,rad,ref} < 26\ °C,$$

$$1 < MR_{ref} < 1.6,$$

$$0.8 < CL_{ref} < 1.5,$$

and/or

$$12 < RR_{ref} < 15,$$

where

$T_{cabin,air,ref}$ = reference vehicle cabin air temperature,
$T_{cabin,rad,ref}$ = reference vehicle cabin surface temperature,
$MR_{ref}$ = passenger metabolic rate reference value,
$CL_{ref}$ = passenger clothing level reference value, and
$RR_{ref}$ = passenger respiratory rate reference value.

The condition value evaluation device 30 is configured to evaluate a passenger condition value CV based on the function:

$$CV = a \cdot (T_{cabin,air} - T_{cabin,air,ref}) + b \cdot (T_{cabin,rad} - T_{cabin,rad,ref}) + c \cdot TBS +$$

$$d \cdot (MR - MR_{ref}) + e \cdot (CL - CL_{ref}) + f \cdot (RR - RR_{ref}) + g \cdot MS$$

where:

CV = passenger condition value,
$T_{cabin,air}$ = vehicle cabin air temperature parameter,
$T_{cabin,air,ref}$ = reference vehicle cabin air temperature parameter,
$T_{cabin,rad}$ = vehicle cabin surface temperature parameter,
$T_{cabin,rad,ref}$ = reference vehicle cabin surface temperature parameter,
TBS = passenger thermal body state parameter,
MR = passenger metabolic rate parameter,
$MR_{ref}$ = passenger metabolic rate reference value,
CL = passenger clothing level parameter,
$CL_{ref}$ = passenger clothing level reference value,
RR = passenger respiratory rate parameter,
$RR_{ref}$ = passenger respiratory rate reference value, and
MS = passenger mood state parameter;
and where

a = vehicle cabin air temperature parameter weighting factor,
b = vehicle cabin surface temperature parameter weighting factor,
c = passenger thermal body state parameter weighting factor,
d = passenger metabolic rate parameter weighting factor,
e = passenger clothing level parameter weighting factor,
f = passenger respiratory rate parameter weighting factor, and
g = passenger mood state parameter weighting factor.

The condition value evaluated by the condition value evaluation device 30 may be provided to a transformation unit 32 for transforming the condition value to an equivalent set temperature or discharge temperature and air distribution setting. Such data from the transformation unit 32 may be used as an input signal for an HVAC control system 34. Alternatively, the condition value evaluated by the condition value evaluation device 30 can be used as an input signal for the cabin air temperature of the vehicle.

The evaluation device 10 may further comprise a condition sensor device 36 for providing measurement data and/or measurement signals to at least one of the parameter evaluation units 14 to 28. The condition sensor device 36 may comprise at least one optical sensor unit 38 for sensing wavelengths in the visible spectrum, and at least one temperature sensor unit 40 for sensing wavelengths in the long-wave infrared spectrum. The optical sensor unit 38 may also be configured for sensing wavelengths in the near infrared spectrum. The optical sensor unit 38 may be equipped with an infrared light source for night vision abilities. The temperature sensor unit 40 may be configured for detecting infrared equivalent blackbody radiation, particularly blackbody radiation around 300 Kelvin.

The pixel density of the optical sensor unit 38 may be at least 4 times higher, preferably at least 5 times higher, than the pixel density of the temperature sensor unit 40. Therewith, the condition sensor device 36 may provide measurement results in sufficient accuracy. At the same time such condition sensor device 36 may be produced with only limited costs, particularly due to the comparatively low pixel density of the temperature sensor unit 40. That is, the resolution provided by the optical sensor unit 38 is much higher than the resolution of the temperature sensor unit 40. Combining the information provided from the two sensor units 38 and 40, in particular by sensor unit fusion, may lead to a significant cost reduction due to a lower pixel count for the temperature sensor unit 40.

As mentioned above, the condition sensor device 36 may provide measurement data and/or measurement signals to at least one of the parameter evaluation units 14 to 28. The different parameter evaluation units 14 to 28 may therefore be configured to evaluate parameters on the basis of specific input data and/or input signals provided by the condition sensor device 36.

The vehicle cabin air temperature parameter evaluation unit 14 may be configured to evaluate a vehicle cabin air temperature parameter $T_{cabin,air}$ based on a measured vehicle cabin surface temperature $T_{surf,i}$. The measured vehicle cabin surface temperature $T_{surf,i}$ may be provided by the condition sensor device 36. The vehicle cabin air temperature parameter evaluation unit 14 may particularly be configured to evaluate a vehicle cabin air temperature parameter $T_{cabin,air}$ based on a plurality of temperature measurements $T_{surf,i}$ of different vehicle interior surfaces.

The evaluation of the vehicle cabin air temperature parameter $T_{cabin,air}$ by the vehicle cabin air temperature parameter evaluation unit 14 may be based on the function:

$$T_{cabin,air} = \sum_{i=1}^{n} w_i \cdot T_{surf,i}$$

with

$$0 < w_i < 1$$

and

$$\sum w_i = 1$$

and where

$T_{cabin,air}$ = vehicle cabin air temperature parameter,
$T_{surf,i}$ = measured vehicle cabin surface temperature, and
$w_i$ = weighting factor for a vehicle interior surface.

The vehicle cabin surface temperature parameter evaluation unit 16 may be configured to evaluate a vehicle cabin surface temperature parameter $T_{cabin,rad}$ based on at least one measured vehicle cabin surface temperature $T_{surf,i}$. The measured vehicle cabin surface temperature $T_{surf,i}$ may be provided by the condition sensor device 36. The vehicle cabin surface temperature parameter evaluation unit 16 may particularly be configured to evaluate a vehicle cabin surface temperature parameter $T_{cabin,rad}$ based on a plurality of temperature measurements $T_{surf,i}$ of different vehicle interior surfaces, such as a dashboard, a ceiling portion, a window portion, and/or a door portion. The vehicle cabin surface temperature parameter $T_{cabin,rad}$ may, for example, be and/or represent a solar and/or radiant flux parameter.

The evaluation of the vehicle cabin surface temperature parameter $T_{cabin,rad}$ by the vehicle cabin surface temperature parameter evaluation unit 16 may be based on the function:

$$T_{cabin,rad} = \sum_{i=1}^{n} w_i \cdot T_{surf,i};$$

with

$$0 < w_i < 1$$

and

$$\sum w_i = 1$$

and where

$T_{cabin,rad}$ = vehicle cabin surface temperature parameter,
$T_{surf,i}$ = measured vehicle cabin surface temperature, and
$w_i$ = weighting factor for a vehicle interior surface.

The thermal body state parameter evaluation unit 18 may be configured to evaluate a thermal body state parameter TBS based on at least one measured skin temperature $T_{skin,i}$. The measured skin temperature $T_{skin,i}$ may be provided by the condition sensor device 36. In particular, the thermal body state parameter evaluation unit 18 may be configured to evaluate a thermal body state parameter TBS based on a weighted average of skin temperature measurements $T_{skin,i}$ as well as a temporal gradient $dT_{skin,i}$ of the skin temperature, preferably a temporal gradient of the skin temperature in the facial area of the passenger.

The thermal body state parameter evaluation unit 18 may be configured to evaluate a thermal body state parameter TBS based on the function:

$$\text{TBS} = a_i \cdot \left( T_{skin,i} - T_{skin,ref} \right) + b_i \cdot \frac{dT_{skin,i}}{dt}$$

with

$$0.2 < a_i < 2$$

$$2 < b_i < 40$$

$$29\ ^\circ C < T_{skin,ref} < 35 ^\circ C,$$

and

$$T_{skin,i} \text{ in } ^\circ C;$$

where

TBS = passenger thermal body state parameter,
$T_{skin,i}$ = measured passenger skin temperature,
Tskin,ref = passenger skin reference temperature.

The metabolic rate parameter evaluation unit 20 may be configured to evaluate a metabolic rate parameter MR based on an evaluated heart beat rate HR or heart beat rate parameter HR. For evaluating a heart beat rate HR or heart beat rate parameter HR, the parameter evaluating device 12 may comprise the heart beat rate parameter evaluation unit 28. The heart beat rate parameter evaluation unit 28 may be configured for providing an evaluated heart beat rate HR or heart beat rate parameter HR to the metabolic rate parameter evaluation unit 20.

The heart beat rate parameter evaluation unit 28 may be configured to evaluate a heart beat rate HR and/or heart beat rate parameter HR based on measurements 42 and/or monitoring results 44 of a vehicle passenger forehead, preferably by measurement of temperature fluctuations caused by the variations in blood flow through the hypodermic blood vessels and/or by measurements of color variations of a vehicle passenger forehead skin. The vehicle passenger forehead measurements 42 and/or monitoring results 44 may be provided by the condition sensor device 36. Measurements 42 and/or monitoring results 44 may also be provided by wearable devices. Such wearable devices may, for example, be smartwatches, wearable heart beat rate measuring devices, wearable electrocardiography devices, pulse oximetry devices, and/or devices for the measurement of oxygen saturation.

The clothing level parameter evaluation unit 22 may be configured to evaluate a clothing level parameter CL based on at least one measured passenger body surface temperature $T_{body\_surf,i}$, particularly a temperature measurement of a local clothing surface in the region of interest of a clothed body part, preferably of a chest and/or arm portion. The clothing level parameter evaluation unit 22 may also be configured to evaluate a clothing level parameter CL based on at least one measured skin temperature $T_{skin,i}$ and/or at least one measured cabin air temperature $T_{cabin,air}$. The measured passenger body surface temperature $T_{body\_surf,i}$, the measured skin temperature $T_{skin,i}$ and/or the at least one measured cabin air temperature $T_{cabin,air}$ may be provided by the condition sensor device 36.

The clothing level parameter evaluation unit 22 may be configured to evaluate a clothing level parameter (CL) based on the function:

$$CL = ((z_1 \cdot T_{cabin,air})^2 + z_2 \cdot T_{cabin,air} + z_3) \cdot T_{body\_surf,i} + z_4 \cdot T_{cabin,air}^2 + z_5 \cdot T_{cabin,air} + z_6$$

where:

CL = passenger clothing level parameter,
$T_{cabin,air}$ = vehicle cabin air temperature parameter,
$T_{body\_surf,i}$ = measured passenger body surface temperature,
and
$z_1$ to $z_6$ = coefficients.

The respiratory rate parameter evaluating unit 24 may be configured to evaluate a respiratory rate parameter RR based on measured temperature fluctuations 46 in the sub-nasal area of the face of a vehicle passenger and/or based on temperature fluctuations 46 caused by alternating airflow through the passenger nose caused by respiratory activity. The measured temperature fluctuations 46 may be provided by the condition sensor device 36.

The mood state parameter evaluation unit 26 may be configured to evaluate a mood state parameter MS based on optical tracking VIS of facial landmarks, preferably eyes, eyebrows, nose and/or mouth of a vehicle passenger, and/or based on a transformation of relative geometric positions of tracked facial landmarks. The input data and/or signals for the mood state parameter evaluation unit 26 may be provided by the condition sensor device 36.

The parameter evaluation device 12 and/or the condition value evaluation device 30 may be provided in the form of a single control device or multiple control devices. Such a control device may comprise a CPU and a data storage device. The data storage may comprise suitable programming code, particularly for providing the required functionality of the controller. The different parameter evaluation units 14 to 28 may be provided by different portions of a programming code or software portions. It is also possible that the different parameter evaluation units 14 to 28 are provided by physically distinct controllers.

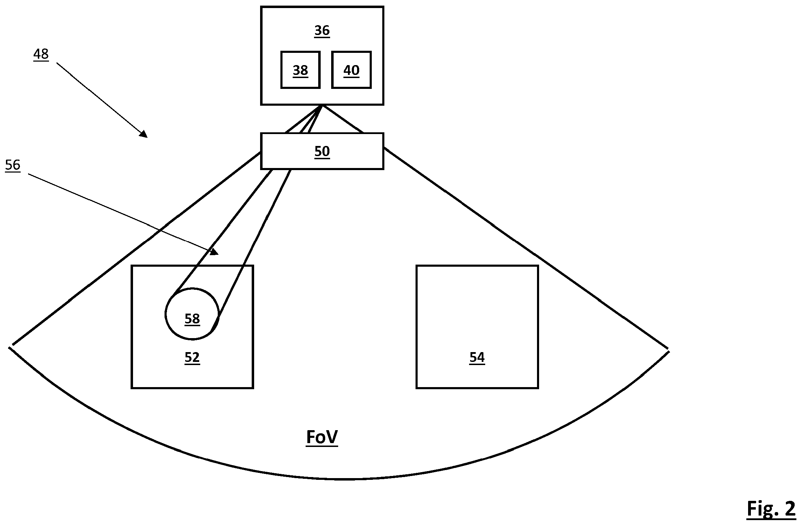

Fig. 2 shows a schematic diagram of a condition sensor device 36 arranged within a passenger cabin 48 of a vehicle. The condition sensor device 36 may, for example, be arranged at or adjacent to a rearview mirror 50. Accordingly, both the optical sensor unit 38 for sensing wavelengths in the visible spectrum, and the temperature sensor unit 40 for sensing wavelengths in the long-wave infrared spectrum may be arranged at the same place within the passenger cabin 48 or at least directly adjacent to each other. It is possible that the two sensor units 38 and 40 are integrated to one single sensor unit. It is also possible that the different sensor units 38 and 40 are located at different positions. In the latter case, the signals from the sensor units 38 and 40 have to be matched in view of the difference in position. This matching may be conducted by a controller, preferably by the parameter evaluation device 12.

As may be comprehended from Fig. 2, the condition sensor device 36 may have a field of view FoV, which covers at least a driver seat 52 and or a co-driver seat 54. The sensor field of view FoV may also cover rear seats of the passenger cabin 48, which are not shown here. A partial view angle 56 of the field of view FoV may cover a driver head position.

The resulting pixel density of an object to be optically sensed by the condition sensor device 36 is dependent on several factors, such as the sensor resolution, the field of view FoV, as well as the position of the condition sensor device 36 relative to the object to be sensed, for example, the head and/or face of a vehicle passenger.

The condition sensor device 36 may have minimal requirements in terms of required pixel densities for the optical sensor unit 38 (visible domain) and for the temperature sensor unit 40 (thermal domain) to be applied for a specific configuration inside a vehicle cabin. The condition sensor device 36 may particularly require a resolution high enough in the visible domain via the optical sensor unit 38 to reliably identify and/or track different parts of the body of a vehicle passenger, particularly the face including the forehead and/or other regions of the face, such as the nose, the cheeks, the temples, the chin and/or the jaw. A minimum pixel density of the temperature sensor unit 40 (thermal domain) may be required to accurately correlate temperatures to the body parts of the vehicle passenger, such as different parts of the face, to the identified regions.

As mentioned above, the pixel density of the optical sensor unit 38 may at least be 4 times higher, preferably at least 5 times higher, than the pixel density of the temperature sensor unit 40. Furthermore, the minimum pixel density of the temperature sensor unit 40 may be defined by the function:

$$\frac{pixel}{deg}\,|\min = d\,\frac{\pi}{180°}\,\frac{\text{HP}}{\text{WF}}$$

where

    d = distance between the temperature sensor and a passenger face,
    HP = minimum number of horizontal pixels for a face resolution, and
    WF = average width of a passenger face.

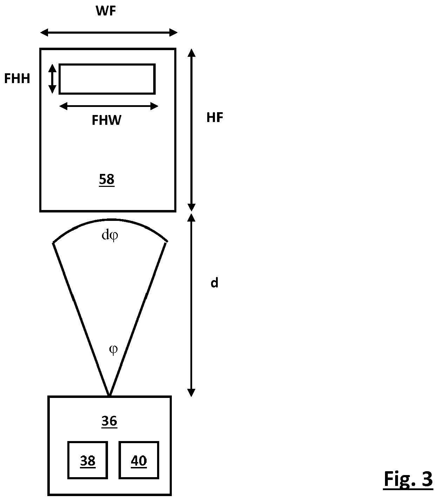

Fig. 3 shows a schematic diagram of a condition sensor device 36 positioned at a distance d from a passenger face 58. The passenger face 58 may have a face width WF and a face height HF. It may furthermore be comprehended from Fig. 3 the forehead width FHW and the forehead height FHH.

Depending on the design of the respective passenger cabin 48, a distance d between the face 58 of vehicle passenger and the condition sensor device 36, particularly the temperature sensor unit 40, may amount to about 500 mm. An average face width WF may amount to about 120 mm.

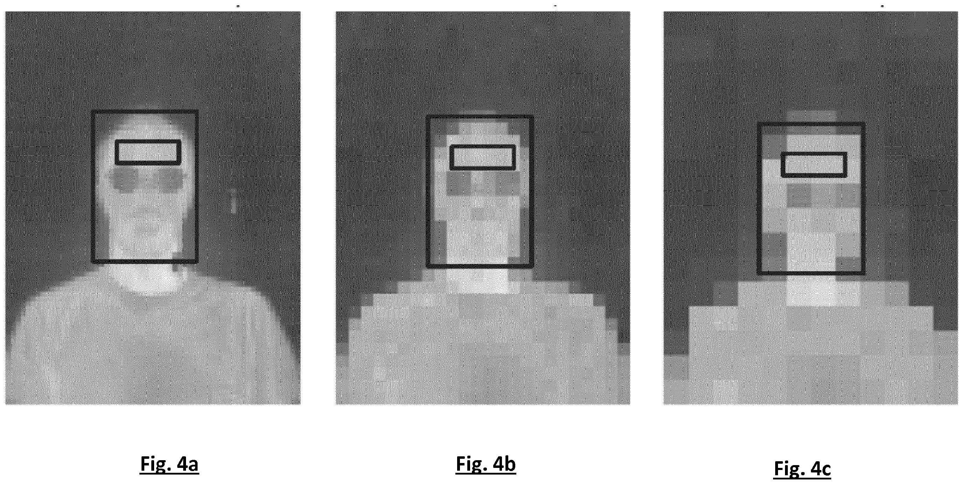

Fig. 4a to 4c temperature measurements of a face with different face resolutions. That is, Fig. 4a shows a temperature measurement of a person with a face resolution of 30x20 pixels, Fig. 4b shows a temperature measurement of a person with a face resolution of 12x8 pixels, and Fig. 4c shows a temperature measurement of a person with a face resolution of 6x4 pixels. It may be comprehended that the resolution according to Fig. 4a is relatively high and allows an accurate temperature correlation to the body parts of the vehicle passenger, but may require a high cost effort for the temperature sensor unit 40. It may furthermore be comprehended that the resolution according to Fig. 4c is relatively low and would not allow any accurate temperature correlation to the body parts of the vehicle passenger, particularly considering the count of only two pixels for the forehead width FHW. Finally, it may be comprehended that the resolution according to Fig. 4b is average and still allows a sufficiently accurate temperature correlation to the body parts of the vehicle passenger, particularly considering the count of four pixels for the forehead width FHW. At the same time, such resolution would require only a limited cost effort for the temperature sensor unit 40. Thus, the face resolution of 12x8 pixels may be sufficient for the shown example.

Following the above, the minimum pixel density of the temperature sensor unit 40 may be defined by:

$$\frac{pixel}{deg}\,|\min = 500\;mm\,\frac{\pi}{180°}\,\frac{8}{120\text{mm}} = 0{,}58\,\frac{pixel}{deg}$$

where

    500 mm = distance between the temperature sensor and a passenger face,
    8 = minimum number of horizontal pixels for a face resolution, and
    120 mm = average width of a passenger face.

In addition to the calculation example above, a preferable range of pixel per deg may generally be between 0.05 and 10 in horizontal direction.

A vehicle with a passenger cabin 48 and a condition sensor device 36 with an according resolution may particularly provide sufficiently accurate measurement results, and may at the same time be provided with only limited costs.

## Claims

1. Evaluation system (10) for the condition evaluation of a vehicle passenger, the system comprising:

    - a parameter evaluation device (12) being configured to evaluate a plurality of parameters of a set of vehicle interior and vehicle passenger parameters, the set of parameters including:

- a vehicle cabin air temperature parameter ($T_{cabin,air}$),
- a vehicle cabin surface temperature parameter ($T_{cabin,rad}$),
- a passenger thermal body state parameter (TBS),
- a passenger metabolic rate parameter (MR),
- a passenger clothing level parameter (CL),
- a passenger respiratory rate parameter (RR), and
- a passenger mood state parameter (MS); and

- a condition value evaluation device (30) being configured to evaluate a passenger condition value (CV) based on the parameters evaluated by the parameter evaluation unit (12);
- wherein the condition value evaluation device (30) is configured to apply a weighting factor for a parameter evaluated by the parameter evaluation device (12); the weighting factor being within one of the ranges of:

$$-10 < a < 10,$$

$$-10 < b < 10,$$

$$0 < c < 8,$$

$$0 < d < 8,$$

$$0 < e < 5,$$

$$-1 < f < 0,$$

and

$$0.2 < g < 3;$$

where:

a = vehicle cabin air temperature parameter weighting factor,
b = vehicle cabin surface temperature parameter weighting factor,
c = passenger thermal body state parameter weighting factor,
d = passenger metabolic rate parameter weighting factor,
e = passenger clothing level parameter weighting factor,
f = passenger respiratory rate parameter weighting factor, and
g = passenger mood state parameter weighting factor

**characterized in that** the condition value evaluation device (30) is configured to evaluate a passenger condition value (CV) based on the function:

$$CV = a \cdot (T_{cabin,\,air} - T_{cabin,air,ref}) + b \cdot (T_{cabin,\,rad} - T_{cabin,rad,ref}) + c \cdot TBS +$$
$$d \cdot (MR - MR_{ref}) + e \cdot (CL - CL_{ref}) + f \cdot (RR - RR_{ref}) + g \cdot MS$$

where:

CV = passenger condition value,
$T_{cabin,air,ref}$ = reference vehicle cabin air temperature parameter,
$T_{cabin,rad,ref}$ = reference vehicle cabin surface temperature parameter,
$MR_{ref}$ = passenger metabolic rate reference value,
$CL_{ref}$ = passenger clothing level reference value,
$RR_{ref}$ = passenger respiratory rate reference value.

2. Evaluation system (10) according to claim 1, wherein the condition value evaluation device (30) is configured to provide the evaluated condition value to a heating ventilation and air conditioning system (HVAC) and/or to an electronic automatic temperature control system (EATC) and/or to an infotainment and/or communication system of a vehicle.

3. Evaluation system (10) according to claim 1 or 2, wherein the condition value evaluation device (30) is configured to evaluate a passenger condition value (CV) based on a plurality of remaining parameters evaluated by the parameter evaluation unit in case of any missing parameter or any parameter evaluation malfunction and wherein the condition value evaluation device (30) is configured to apply a predefined substitute parameter value for any missing parameter or in case of any parameter evaluation malfunction and wherein the condition value evaluation device (30) is configured to apply a weighting factor for each substitute parameter value, said weighting factor corresponding to the weighting factor of the substituted parameter.

4. Evaluation system (10) according to any one of the preceding claims, wherein the condition value evaluation device (30) is configured to evaluate a passenger condition value (CV) based on at least one predefined parameter reference value, the reference value being within one of the ranges of:

$$16\ °C < T_{cabin,air,ref} < 26\ °C,$$

$$16\ °C < T_{cabin,rad,ref} < 26\ °C,$$

$$1 < MR_{ref} < 1.6,$$

$$0.8 < CL_{ref} < 1.5,$$

and/or

$$12 < RR_{ref} < 15,$$

where

$T_{cabin,air,ref}$ = reference vehicle cabin air temperature,
$T_{cabin,rad,ref}$ = reference vehicle cabin surface temperature,
$MR_{ref}$ = passenger metabolic rate reference value,
$CL_{ref}$ = passenger clothing level reference value, and
$RR_{ref}$ = passenger respiratory rate reference value.

5. Evaluation system (10) according to any one of the preceding claims, wherein the parameter evaluation device (12) is configured to evaluate a thermal body state parameter (TBS), a metabolic rate parameter (MR), a clothing level parameter (CL) and/or a respiratory rate parameter (RR) based on at least one computer vision algorithm and/or based on at least one measurement of a passenger's head and/or other body parts, preferably an arm, chest and/or a hand.

6. Evaluation system (10) according to any one of the preceding claims, wherein the parameter evaluation device (12) is configured to evaluate a thermal body state parameter (TBS) based on at least one measured skin temperature in a region of interest, preferably of an unclothed body part and/or of a facial, arm and/or hand portion of a vehicle passenger and/or of a body part exposed to solar radiation, and/or wherein the parameter evaluation device is configured to evaluate a local and/or a global thermal body state parameter.

7. Evaluation system (10) according to any one of the preceding claims, wherein the parameter evaluation device (12) is configured to evaluate a thermal body state parameter (TBS) based on a weighted average of skin temperature measurements as well as a temporal gradient of the skin temperature, preferably a temporal gradient of the skin temperature in the facial area of the passenger, and/or wherein the parameter evaluation device (12) is configured to evaluate a thermal body state parameter (TBS) based on the function:

$$\text{TBS} = a_i \cdot \left(T_{skin,\,i} - T_{skin,ref}\right) + b_i \cdot \frac{dT_{skin,\,i}}{dt}$$

with

$$0.2 < a_i < 2$$

$$2 < b_i < 40$$

$$29\ °C < T_{skin,ref} < 35°C,$$

and

$$T_{skin,i} \text{ in } °C;$$

where

TBS = passenger thermal body state parameter,
$T_{skin,i}$ = measured passenger skin temperature,
Tskin,ref = passenger skin reference temperature.

8. Evaluation system (10) according to any one of the preceding claims, wherein the parameter evaluation device (12) is configured to evaluate a clothing level parameter (CL) based on at least one temperature measurement of a local clothing surface in the region of interest of a clothed body part, preferably of a chest and/or arm portion, and/or wherein the parameter evaluation device (12) is configured to evaluate a clothing level parameter (CL) based on the function:

$$\text{CL} = \left(\sum_{i=0}^{n} z_i T_{Cabin,air}^{i}\right)\left(1 + T_{body\_surf_j}\right) + \sum_{i=0}^{m} c_i T_{body\_surf}^{i}$$

and/or based on the function:

$$\text{CL} = \left((z_1 \cdot T_{cabin,air})^2 + z_2 \cdot T_{cabin,air} + z_3\right) \cdot T_{body\_surf,i} + z_4 \cdot T_{cabin,air}^{2} + z_5 \cdot T_{cabin,air} + z_6$$

where:

CL = passenger clothing level parameter,
$T_{cabin,air}$ = vehicle cabin air temperature parameter,
$T_{body\_surf,i}$ = measured passenger body surface temperature,

and

$z_i$; $z_1$ to $z_6$ = coefficients.

9. Evaluation system (10) according to any one of the preceding claims, wherein the parameter evaluation device (12) is configured to evaluate a respiratory rate parameter (RR) based on measured temperature fluctuations in the sub-nasal area of the face of a vehicle passenger and/or based on temperature fluctuations caused by alternating airflow through the passenger nose caused by respiratory activity and/or wherein the parameter evaluation device (12) is configured to evaluate a respiratory rate parameter (RR) using a band pass filter and/or a Fast-Fourier-Transformation.

10. Evaluation system (10) according to any one of the preceding claims, wherein the parameter evaluation device (12) is configured to evaluate a metabolic rate parameter (MR) based on an evaluated heart beat rate and/or heart beat rate parameter (HR) and/or wherein the parameter evaluation device (12) is configured to evaluate a heart beat rate (HR) and/or heart beat rate parameter (HR) based on measurements and/or monitoring results of a vehicle passenger forehead, preferably by measurement of temperature fluctuations caused by the variations in blood flow through the hypodermic blood vessels and/or by measurements with a framerate of at least 2 fps, more preferably at least 3 fps, and/or by measurements of color variations of a vehicle passenger forehead skin, preferably magnified by Eulerian Video Magnification algorithms, and/or the parameter evaluation device is configured to evaluate a metabolic rate parameter (MR) using a band pass filter and/or a Fast-Fourier- Transformation.

11. Evaluation system (10) according to any one of the preceding claims, wherein the parameter evaluation device (12) is configured to evaluate a mood state parameter (MS) based on optical tracking of facial landmarks, preferably eyes, eyebrows, nose and/or mouth of a vehicle passenger, and/or based on a transformation of relative geometric positions of tracked facial landmarks.

12. Evaluation system (10) according any one of the preceding claims 1 to 11, comprising a condition sensor device (36), the condition sensor device (36) comprising:

- at least one optical sensor unit (38) for sensing wavelength in the visible spectrum, and
- at least one temperature sensor unit (40) for sensing wavelengths in the long-wave infrared spectrum,
- wherein the pixel density of the optical sensor unit (38) is higher than the pixel density of the temperature sensor unit (40).

13. Evaluation system (10) according to claim 12, wherein the minimum pixel density of the temperature sensor unit (40) is defined by the function:

$$\frac{pixel}{deg} \;\Big|\min = d \;\frac{\pi}{180°}\frac{\text{HP}}{\text{WF}}$$

where

d = distance between the temperature sensor and a passenger face,
HP = minimum number of horizontal pixels for a face resolution, and
WF = average width of a passenger face.

14. Method for evaluating the condition of a vehicle passenger with an evaluation system (10) according to any one of claims 1 to 13 the method comprising the steps of:

- evaluating a plurality of parameters of a set of vehicle interior and/or vehicle passenger parameters, the set of parameters including:

- a vehicle cabin air temperature parameter ($T_{cabin,air}$),
- a vehicle cabin surface temperature parameter ($T_{cabin,rad}$),
- a passenger thermal body state parameter (TBS),
- a passenger metabolic rate parameter (MR),
- a passenger clothing level parameter (CL),
- a passenger respiratory rate parameter (RR), and

- a passenger mood state parameter (MS); and

- evaluating a passenger condition value (CV) based on the plurality of evaluated parameters;
- wherein the evaluation of the condition value (CV) is conducted by applying a weighting factor for an evaluated parameter; the weighting factor being within one of the ranges of:

$$-10 < a < 10,$$

$$-10 < b < 10,$$

$$0 < c < 8,$$

$$0 < d < 8,$$

$$0 < e < 5,$$

$$-1 < f < 0,$$

and

$$0.2 < g < 3;$$

where:

a = vehicle cabin air temperature parameter weighting factor,
b = vehicle cabin surface temperature parameter weighting factor,
c = passenger thermal body state parameter weighting factor,
d = passenger metabolic rate parameter weighting factor,
e = passenger clothing level parameter weighting factor,
f = passenger respiratory rate parameter weighting factor, and
g = passenger mood state parameter weighting factor

**characterized in that** a passenger condition value (CV) is evaluated based on the function:

$$CV = a \cdot (T_{cabin,air} - T_{cabin,air,ref}) + b \cdot (T_{cabin,rad} - T_{cabin,rad,ref}) + c \cdot TBS +$$
$$d \cdot (MR - MR_{ref}) + e \cdot (CL - CL_{ref}) + f \cdot (RR - RR_{ref}) + g \cdot MS$$

where:

CV = passenger condition value,
$T_{cabin,air,ref}$ = reference vehicle cabin air temperature parameter,
$T_{cabin,rad,ref}$ = reference vehicle cabin surface temperature parameter,
$MR_{ref}$ = passenger metabolic rate reference value,
$CL_{ref}$ = passenger clothing level reference value,
$RR_{ref}$ = passenger respiratory rate reference value.

**Patentansprüche**

1. Bewertungssystem (10) für die Bewertung des Zustands eines Fahrzeuginsassen, wobei das System umfasst:

- eine Parameterbewertungsvorrichtung (12), die dafür ausgelegt ist, mehrere Parameter eines Satzes von Fahrzeuginnenraum- und Fahrzeuginsassenparametern zu bewerten, wobei der Satz von Parametern umfasst:

- einen Parameter für die Lufttemperatur im Fahrzeuginnenraum ($T_{cabin,air}$),
- einen Parameter für die Oberflächentemperatur im Fahrzeuginnenraum ($T_{cabin,rad}$),
- einen Parameter für den thermischen Körperzustand des Insassen (TBS),
- einen Parameter für die Stoffwechselrate des Insassen (MR),
- einen Parameter für den Bekleidungsgrad des Insassen (CL),
- einen Parameter für die Atemfrequenz des Insassen (RR), und
- einen Parameter für den Gemütszustand des Insassen (MS); und

- eine Zustandswert-Bewertungsvorrichtung (30), die dafür ausgelegt ist, einen Insassenzustandswert (CV) basierend auf den von der Parameterbewertungseinheit (12) bewerteten Parametern zu bewerten;
- wobei die Zustandswert-Bewertungsvorrichtung (30) dafür ausgelegt ist, einen Gewichtungsfaktor für einen von der Parameterbewertungsvorrichtung (12) bewerteten Parameter anzuwenden; wobei der Gewichtungsfaktor innerhalb eines der folgenden Bereiche liegt:

$$-10 < a < 10,$$

$$-10 < b < 10,$$

$$0 < c < 8,$$

$$0 < d < 8,$$

$$0 < e < 5,$$

$$-1 < f < 0,$$

und

$$0{,}2 < g < 3;$$

wobei:

a = Gewichtungsfaktor des Parameters für die Lufttemperatur im Fahrzeuginnenraum,
b = Gewichtungsfaktor des Parameters für die Oberflächentemperatur im Fahrzeuginnenraum,
c = Gewichtungsfaktor des Parameters für den thermischen Körperzustand des Insassen,
d = Gewichtungsfaktor des Parameters für die Stoffwechselrate des Insassen,
e = Gewichtungsfaktor des Parameters für den Bekleidungsgrad des Insassen,
f = Gewichtungsfaktor des Parameters für die Atemfrequenz des Insassen und
g = Gewichtungsfaktor des Parameters für den Gemütszustand des Insassen

**dadurch gekennzeichnet, dass** die Zustandswert-Bewertungsvorrichtung (30) dafür ausgelegt ist, einen Insassenzustandswert (CV) basierend auf der folgenden Funktion zu bewerten:

$$CV = a \cdot (T_{cabin,\,air} - T_{cabin,air,ref}) + b \cdot (T_{cabin,\,rad} - T_{cabin,rad,ref}) + c \cdot TBS +$$
$$d \cdot (MR - MR_{ref}) + e \cdot (CL - CL_{ref}) + f \cdot (RR - RR_{ref}) + g \cdot MS$$

wobei:

CV = Insassenzustandswert,
$T_{cabin,air,ref}$ = Referenzwert des Parameters für die Lufttemperatur im Fahrzeuginnenraum,
$T_{cabin,rad,ref}$ = Referenzwert des Parameters für die Oberflächentemperatur im Fahrzeuginnenraum,
$MR_{ref}$ = Referenzwert für die Stoffwechselrate des Insassen,
$CL_{ref}$ = Referenzwert für den Bekleidungsgrad des Insassen,
$RR_{ref}$ = Referenzwert für die Atemfrequenz des Insassen.

2. Bewertungssystem (10) gemäß Anspruch 1, wobei die Zustandswert-Bewertungsvorrichtung (30) dafür ausgelegt ist, den bewerteten Zustandswert an ein Heizungs-, Lüftungs- und Klimasystem (HVAC) und/oder an ein elektronisches automatisches Temperaturregelungssystem (EATC) und/oder an ein Infotainment- und/oder Kommunikationssystem eines Fahrzeugs bereitzustellen.

3. Bewertungssystem (10) gemäß Anspruch 1 oder 2, wobei die Zustandswert-Bewertungsvorrichtung (30) dafür ausgelegt ist, im Falle eines fehlenden Parameters oder einer Fehlfunktion der Parameterbewertung einen Insassenzustandswert (CV) basierend auf mehreren verbleibenden, von der Parameterbewertungseinheit bewerteten Parametern zu bewerten, und wobei die Zustandswert-Bewertungsvorrichtung (30) dafür ausgelegt ist, einen vordefinierten Ersatzparameterwert für jeden fehlenden Parameter oder im Falle einer Fehlfunktion der Parameterbewertung anzuwenden, und wobei die Zustandswert-Bewertungsvorrichtung (30) dafür ausgelegt ist, einen Gewichtungsfaktor für jeden Ersatzparameterwert anzuwenden, wobei der Gewichtungsfaktor dem Gewichtungsfaktor des ersetzten Parameters entspricht.

4. Bewertungssystem (10) gemäß einem der vorstehenden Ansprüche, wobei die Zustandswert-Bewertungsvorrichtung (30) dafür ausgelegt ist, einen Insassenzustandswert (CV) basierend auf wenigstens einem vordefinierten Parameter-Referenzwert zu bewerten, wobei der Referenzwert innerhalb eines der folgenden Bereiche liegt:

$$16\ °C < T_{cabin,air,ref} < 26\ °C,$$

$$16\ °C < T_{cabin,rad,ref} < 26\ °C,$$

$$1 < MR_{ref} < 1,6,$$

$$0,8 < CL_{ref} < 1,5,$$

und/oder

$$12 < RR_{ref} < 15,$$

wobei

$T_{cabin,air,ref}$ = Referenzwert des Parameters für die Lufttemperatur im Fahrzeuginnenraum,
$T_{cabin,rad,ref}$ = Referenzwert des Parameters für die Oberflächentemperatur im Fahrzeuginnenraum,
$MR_{ref}$ = Referenzwert für die Stoffwechselrate des Insassen,
$CL_{ref}$ = Referenzwert für den Bekleidungsgrad des Insassen,
$RR_{ref}$ = Referenzwert für die Atemfrequenz des Insassen.

5. Bewertungssystem (10) gemäß einem der vorstehenden Ansprüche, wobei die Parameterbewertungsvorrichtung (12) dafür ausgelegt ist, einen Parameter für den thermischen Körperzustand (TBS), einen Parameter für die Stoffwechselrate (MR), einen Parameter für den Bekleidungsgrad (CL) und/oder einen Parameter für die Atemfrequenz (RR) basierend auf wenigstens einem Computersehen-Algorithmus und/oder basierend auf wenigstens einer Messung am Kopf und/oder an anderen Körperteilen, vorzugsweise am Arm, an der Brust und/oder einer Hand, eines Insassen zu bewerten.

6. Bewertungssystem (10) gemäß einem der vorstehenden Ansprüche, wobei die Parameterbewertungsvorrichtung (12) dafür ausgelegt ist, einen Parameter für den thermischen Körperzustand (TBS) basierend auf wenigstens einer gemessenen Hauttemperatur in einem interessierenden Bereich, vorzugsweise eines unbekleideten Körperteils und/oder eines Gesichts-, Arm- und/oder Handbereichs eines Fahrzeuginsassen und/oder eines Körperteils, der Sonnenstrahlung ausgesetzt ist, zu bewerten, und/oder wobei die Parameterbewertungsvorrichtung dafür ausgelegt ist, einen lokalen und/oder einen globalen Parameter für den thermischen Körperzustand zu bewerten.

7. Bewertungssystem (10) gemäß einem der vorstehenden Ansprüche, wobei die Parameterbewertungsvorrichtung (12) dafür ausgelegt ist, einen Parameter für den thermischen Körperzustand (TBS) basierend auf einem gewichteten Mittelwert von Hauttemperaturmessungen sowie einem zeitlichen Gradienten der Hauttemperatur, vorzugsweise einem zeitlichen Gradienten der Hauttemperatur im Gesichtsbereich des Insassen, zu bewerten, und/oder wobei die Parameterbewertungsvorrichtung (12) dafür ausgelegt ist, einen Parameter für den thermischen Körperzustand (TBS) basierend auf der folgenden Funktion zu bewerten:

$$\text{TBS} = a_i \cdot \left( T_{skin,i} - T_{skin,ref} \right) + b_i \cdot \frac{dT_{skin,i}}{dt}$$

mit

$$0,2 < a_i < 2$$

$$2 < b_i < 40$$

$$29\ {}^{\circ}C < T_{skin,ref} < 35\ {}^{\circ}C,$$

und

$$T_{skin,i}\ \text{in}\ {}^{\circ}C;$$

wobei

TBS = Parameter für den thermischen Körperzustand des Insassen,
$T_{skin,i}$ = gemessene Hauttemperatur des Insassen,
$T_{skin,ref}$ = Referenz-Hauttemperatur des Insassen.

8. Bewertungssystem (10) gemäß einem der vorstehenden Ansprüche, wobei die Parameterbewertungsvorrichtung (12) dafür ausgelegt ist, einen Parameter für den Bekleidungsgrad (CL) basierend auf wenigstens einer Temperaturmessung einer lokalen Bekleidungsoberfläche im interessierenden Bereich eines bekleideten Körperteils, vorzugsweise eines Brust- und/oder Armabschnitts, zu bewerten, und/oder wobei die Parameterbewertungsvorrichtung (12) dafür ausgelegt ist, einen Parameter für den Bekleidungsgrad (CL) basierend auf der folgenden Funktion zu bewerten:

$$\text{CL} = \left( \sum_{i=0}^{n} z_i T_{Cabin,air}^{i} \right)\left(1 + T_{body\_surf_j}\right) + \sum_{i=0}^{m} c_i T_{body\_surf}^{i}$$

und/oder basierend auf der Funktion:

$$\mathrm{CL} = ((z_1 \cdot T_{cabin,\,air})^2 + z_2 \cdot T_{cabin,\,air} + z_3) \cdot T_{body\_surf,\,i} + z_4 \cdot T_{cabin,\,air}{}^2 + z_5$$
$$\cdot\, T_{cabin,\,air} + z_6$$

wobei:

CL = Parameter für den Bekleidungsgrad des Insassen,
$T_{cabin,air}$ = Parameter für die Lufttemperatur im Fahrzeuginnenraum,
$T_{body\_surf,i}$ = gemessene Körperoberflächentemperatur des Insassen, und
$z_i$; $z_1$ bis $z_6$ = Koeffizienten.

9. Bewertungssystem (10) gemäß einem der vorstehenden Ansprüche, wobei die Parameterbewertungsvorrichtung (12) dafür ausgelegt ist, einen Parameter für die Atemfrequenz (RR) basierend auf gemessenen Temperaturschwankungen im subnasalen Bereich des Gesichts eines Fahrzeuginsassen und/oder basierend auf Temperaturschwankungen, die durch einen wechselnden Luftstrom durch die Nase des Insassen verursacht werden, der durch die Atmungsaktivität verursacht wird, zu bewerten, und/oder wobei die Parameterbewertungsvorrichtung (12) dafür ausgelegt ist, einen Parameter für die Atemfrequenz (RR) unter Verwendung eines Bandpassfilters und/oder einer Fast-Fourier-Transformation zu bewerten.

10. Bewertungssystem (10) gemäß einem der vorstehenden Ansprüche, wobei die Parameterbewertungsvorrichtung (12) dafür ausgelegt ist, einen Parameter für die Stoffwechselrate (MR) basierend auf einer bewerteten Herzfrequenz und/oder einem Parameter für die Herzfrequenz (HR) zu bewerten, und/oder wobei die Parameterbewertungsvorrichtung (12) dafür ausgelegt ist, eine Herzfrequenz (HR) und/oder einen Parameter für die Herzfrequenz (HR) basierend auf Messungen und/oder Überwachungsergebnissen einer Stirn des Fahrzeuginsassen zu bewerten, vorzugsweise durch Messen von Temperaturschwankungen, die durch Schwankungen des Blutflusses durch die subkutanen Blutgefäße verursacht werden, und/oder durch Messungen mit einer Framerate von wenigstens 2 fps, vorzugsweise wenigstens 3 fps, und/oder durch Messungen von Farbveränderungen der Haut an der Stirn des Fahrzeuginsassen, vorzugsweise vergrößert durch Eulersche Videovergrößerungsalgorithmen, und/oder die Parameterbewertungsvorrichtung dafür ausgelegt ist, einen Parameter für die Stoffwechselrate (MR) unter Verwendung eines Bandpassfilters und/oder einer Fast-Fourier-Transformation zu bewerten.

11. Bewertungssystem (10) gemäß einem der vorstehenden Ansprüche, wobei die Parameterbewertungsvorrichtung (12) dafür ausgelegt ist, einen Parameter für den Gemütszustand (MS) basierend auf der optischen Verfolgung von Gesichtsmerkmalen, vorzugsweise Augen, Augenbrauen, Nase und/oder Mund eines Fahrzeuginsassen, und/oder basierend auf einer Transformation der relativen geometrischen Positionen der verfolgten Gesichtsmerkmale zu bewerten.

12. Bewertungssystem (10) gemäß einem der vorstehenden Ansprüche 1 bis 11, eine Zustandssensorvorrichtung (36) umfassend, wobei die Zustandssensorvorrichtung (36) umfasst:

- wenigstens eine optische Sensoreinheit (38) zum Erfassen der Wellenlänge im sichtbaren Spektrum und
- wenigstens eine Temperatursensoreinheit (40) zum Erfassen von Wellenlängen im langwelligen Infrarotbereich,
- wobei die Pixeldichte der optischen Sensoreinheit (38) höher ist als die Pixeldichte der Temperatursensoreinheit (40).

13. Bewertungssystem (10) gemäß Anspruch 12, wobei die minimale Pixeldichte der Temperatursensoreinheit (40) durch folgende Funktion definiert ist:

$$\frac{pixel}{deg}\,\bigg|\,\min = d\,\frac{\pi}{180°}\,\frac{\mathrm{HP}}{\mathrm{WF}}$$

wobei

d = Abstand zwischen dem Temperatursensor und dem Gesicht eines Insassen,

HP = Mindestanzahl an horizontalen Pixeln für eine Gesichtsauflösung und

WF = durchschnittliche Breite des Gesichts eines Insassen.

**14.** Verfahren für die Bewertung des Zustands eines Fahrzeuginsassen mit einem Bewertungssystem (10) gemäß einem der Ansprüche 1 bis 13, wobei das Verfahren die Schritte umfasst:

- Bewerten mehrerer Parameter eines Satzes von Fahrzeuginnenraum- und/oder Fahrzeuginsassenparametern, wobei der Satz von Parametern umfasst:

      - einen Parameter für die Lufttemperatur im Fahrzeuginnenraum ($T_{cabin,air}$),
      - einen Parameter für die Oberflächentemperatur im Fahrzeuginnenraum ($T_{cabin,rad}$),
      - einen Parameter für den thermischen Körperzustand des Insassen (TBS),
      - einen Parameter für die Stoffwechselrate des Insassen (MR),
      - einen Parameter für den Bekleidungsgrad des Insassen (CL),
      - einen Parameter für die Atemfrequenz des Insassen (RR) und
      - einen Parameter für den Gemütszustand des Insassen (MS); und

- Bewerten eines Insassenzustandswerts (CV) basierend auf den mehreren bewerteten Parametern;
- wobei die Bewertung des Zustandswerts (CV) durch Anwenden eines Gewichtungsfaktors für einen bewerteten Parameter durchgeführt wird; wobei der Gewichtungsfaktor innerhalb eines der folgenden Bereiche liegt:

$$-10 < a < 10,$$

$$-10 < b < 10,$$

$$0 < c < 8,$$

$$0 < d < 8,$$

$$0 < e < 5,$$

$$-1 < f < 0,$$

und

$$0,2 < g < 3;$$

wobei:

      a = Gewichtungsfaktor des Parameters für die Lufttemperatur im Fahrzeuginnenraum,
      b = Gewichtungsfaktor des Parameters für die Oberflächentemperatur im Fahrzeuginnenraum,
      c = Gewichtungsfaktor des Parameters für den thermischen Körperzustand des Insassen,
      d = Gewichtungsfaktor des Parameters für die Stoffwechselrate des Insassen,
      e = Gewichtungsfaktor des Parameters für den Bekleidungsgrad des Insassen,
      f = Gewichtungsfaktor des Parameters für die Atemfrequenz des Insassen und
      g = Gewichtungsfaktor des Parameters für den Gemütszustand des Insassen

**dadurch gekennzeichnet, dass** ein Insassenzustandswert (CV) basierend auf der folgenden Funktion bewertet wird:

$$CV = a \cdot (T_{cabin,\,air} - T_{cabin,air,ref}) + b \cdot (T_{cabin,\,rad} - T_{cabin,rad,ref}) + c \cdot TBS +$$
$$d \cdot (MR - MR_{ref}) + e \cdot (CL - CL_{ref}) + f \cdot (RR - RR_{ref}) + g \cdot MS$$

wobei:

CV = Insassenzustandswert,
$T_{cabin,air,ref}$ = Referenzwert des Parameters für die Lufttemperatur im Fahrzeuginnenraum,
$T_{cabin,rad,ref}$ = Referenzwert des Parameters für die Oberflächentemperatur der Fahrzeugkabine,
$MR_{ref}$ = Referenzwert für die Stoffwechselrate des Insassen,
$CL_{ref}$ = Referenzwert für den Bekleidungsgrad des Insassen,
$RR_{ref}$ = Referenzwert für die Atemfrequenz des Insassen.

## Revendications

1. Système d'évaluation (10) pour l'évaluation de l'état d'un passager de véhicule, le système comprenant :

- un dispositif d'évaluation de paramètres (12) configuré pour évaluer une pluralité de paramètres d'un ensemble de paramètres de l'intérieur du véhicule et du passager de véhicule, l'ensemble de paramètres comprenant :

- un paramètre de température de l'air de l'habitacle du véhicule ($T_{cabin,air}$),
- un paramètre de température de surface de l'habitacle du véhicule ($T_{cabin,rad}$)
- un paramètre d'état corporel thermique du passager (TBS),
- un paramètre de taux métabolique du passager (MR),
- un paramètre de niveau d'habillement du passager (CL)
- un paramètre de fréquence respiratoire du passager (RR), et
- un paramètre d'état d'humeur du passager (MS) ; et

- un dispositif d'évaluation de valeur d'état (30) étant configuré pour évaluer une valeur d'état de passager (CV) sur la base des paramètres évalués par l'unité d'évaluation de paramètres (12) ;
- le dispositif d'évaluation de valeur d'état (30) étant configuré pour appliquer un facteur de pondération pour un paramètre évalué par le dispositif d'évaluation de paramètres (12) ; le facteur de pondération étant dans l'une des plages suivantes :

```
- 10 < a < 10,

- 10 < b < 10,

  0 < c < 8,

  0 < d < 8,

  0 < e < 5,

- 1 < f < 0,
```

et

```
0,2 < g < 3 ;
```

avec :

a = facteur de pondération du paramètre de température de l'air de l'habitacle du véhicule,
b = facteur de pondération du paramètre de température de surface de l'habitacle du véhicule,
c = facteur de pondération du paramètre d'état corporel thermique du passager,
d = facteur de pondération du paramètre de taux métabolique du passager,
e = facteur de pondération du paramètre de niveau d'habillement du passager,
f = facteur de pondération du paramètre de fréquence respiratoire du passager, et
g = facteur de pondération du paramètre d'état d'humeur du passager

**caractérisé en ce que** le dispositif d'évaluation de valeur d'état (30) est configuré pour évaluer une valeur d'état de passager (CV) sur la base de la fonction :

$$CV = a \cdot (T_{cabin,air} - T_{cabin,air,ref}) + b \cdot (T_{cabin,rad} - T_{cabin,rad,ref}) + c \cdot TBS + d \cdot (MR - MR_{ref}) + e \cdot (CL - CL_{ref}) + f \cdot (RR - RR_{ref}) + g \cdot MS$$

avec :

CV = valeur d'état de passager,
$T_{cabin,air,ref}$ = paramètre de température de l'air de l'habitacle du véhicule de référence,
$T_{cabin,rad,ref}$ = paramètre de température de surface de l'habitacle du véhicule de référence,
$MR_{ref}$ = valeur de référence du taux métabolique du passager,
$CL_{ref}$ = valeur de référence du niveau d'habillement du passager,
$RR_{ref}$ = valeur de référence de la fréquence respiratoire du passager.

2. Système d'évaluation (10) selon la revendication 1, le dispositif d'évaluation de valeur d'état (30) étant configuré pour fournir la valeur d'état évaluée à un système de chauffage, ventilation et climatisation (HVAC) et/ou à un système de contrôle électronique automatique de la température (EATC) et/ou à un système d'infodivertissement et/ou de communication d'un véhicule.

3. Système d'évaluation (10) selon la revendication 1 ou 2, le dispositif d'évaluation de valeur d'état (30) étant configuré pour évaluer une valeur d'état de passager (CV) sur la base d'une pluralité de paramètres restants évalués par l'unité d'évaluation de paramètres dans le cas de tout paramètre manquant ou de tout dysfonctionnement d'évaluation de paramètre, et le dispositif d'évaluation de valeur d'état (30) étant configuré pour appliquer une valeur de paramètre de substitution prédéfinie pour tout paramètre manquant ou dans le cas de tout dysfonctionnement d'évaluation de paramètre, et le dispositif d'évaluation de valeur d'état (30) étant configuré pour appliquer un facteur de pondération pour chaque valeur de paramètre de substitution, ledit facteur de pondération correspondant au facteur de pondération du paramètre substitué.

4. Système d'évaluation (10) selon l'une quelconque des revendications précédentes, le dispositif d'évaluation de valeur d'état (30) étant configuré pour évaluer une valeur d'état de passager (CV) sur la base d'au moins une valeur de référence de paramètre prédéfinie, la valeur de référence étant comprise dans l'une des plages suivantes :

$$16 \, °C < T_{cabin,air,ref} < 26 \, °C,$$

$$16 \, °C < T_{cabin,rad,ref} < 26 \, °C,$$

$$1 < MR_{ref} < 1,6,$$

$$0,8 < CL_{ref} < 1,5,$$

et/ou

$$12 < RR_{ref} < 15,$$

avec :

$T_{cabin,air,ref}$ = température de l'air de l'habitacle du véhicule de référence,
$T_{cabin,rad,ref}$ = température de surface de l'habitacle du véhicule de référence,
$MR_{ref}$ = valeur de référence du taux métabolique du passager,
$CL_{ref}$ = valeur de référence du niveau d'habillement du passager, et
$RR_{ref}$ = valeur de référence de la fréquence respiratoire du passager.

**5.** Système d'évaluation (10) selon l'une quelconque des revendications précédentes, le dispositif d'évaluation de paramètres (12) étant configuré pour évaluer un paramètre d'état corporel thermique (TBS), un paramètre de taux métabolique (MR), un paramètre de niveau d'habillement (CL) et/ou un paramètre de fréquence respiratoire (RR) sur la base d'au moins un algorithme de vision par ordinateur et/ou sur la base d'au moins une mesure de la tête et/ou d'autres parties du corps, de préférence un bras, une poitrine et/ou une main d'un passager.

**6.** Système d'évaluation (10) selon l'une quelconque des revendications précédentes, le dispositif d'évaluation de paramètres (12) étant configuré pour évaluer un paramètre d'état corporel thermique (TBS) sur la base d'au moins une température de peau mesurée dans une région d'intérêt, de préférence d'une partie du corps non habillée et/ou d'une partie du visage, d'un bras et/ou d'une main d'un passager de véhicule et/ou d'une partie du corps exposée au rayonnement solaire, et/ou le dispositif d'évaluation de paramètres étant configuré pour évaluer un paramètre d'état corporel thermique local et/ou global.

**7.** Système d'évaluation (10) selon l'une quelconque des revendications précédentes, le dispositif d'évaluation de paramètres (12) étant configuré pour évaluer un paramètre d'état corporel thermique (TBS) sur la base d'une moyenne pondérée de mesures de température de la peau ainsi que d'un gradient temporel de la température de la peau, de préférence un gradient temporel de la température de la peau dans la zone du visage du passager, et/ou le dispositif d'évaluation de paramètres (12) étant configuré pour évaluer un paramètre d'état corporel thermique (TBS) sur la base de la fonction :

$$TBS \ : \ a_i \cdot \left(T_{skin,i} - T_{skin,ref}\right) + b_i \cdot \frac{dT_{skin,i}}{dt}$$

avec

$$0,2 < a_i < 2$$

$$2 < b_i < 40$$

$$29 \ °C < T_{skin,ref} < 35 \ °C,$$

et

$$T_{skin,i} \ en \ °C \ ;$$

avec :

TBS = paramètre d'état corporel thermique du passager,
$T_{skin,i}$ = température mesurée de la peau du passager,
$T_{skin,ref}$ = température de référence de la peau du passager.

8. Système d'évaluation (10) selon l'une quelconque des revendications précédentes, le dispositif d'évaluation de paramètres (12) étant configuré pour évaluer un paramètre de niveau d'habillement (CL) sur la base d'au moins une mesure de température d'une surface d'habillement locale dans la région d'intérêt d'une partie du corps habillée, de préférence d'une portion de poitrine et/ou de bras, et/ou le dispositif d'évaluation de paramètres (12) étant configuré pour évaluer un paramètre de niveau d'habillement (CL) sur la base de la fonction :

$$CL = \left(\sum_{i=0}^{n} z_i T_{Cabin,air}^i\right)\left(1 + T_{body\_surf_j}\right) + \sum_{i=0}^{m} c_i T_{body\_surf}^i$$

et/ou sur la base de la fonction :

$$CL = \left(\left(z_1 \cdot T_{cabin,air}\right)^2 + z_2 \cdot T_{cabin,air} + z_3\right) \cdot T_{body\_surf,i} + z_4 \cdot T_{cabin,air}^2 + z_5 \cdot T_{cabin,air} + z_6$$

avec :

CL = paramètre de niveau d'habillement du passager,
$T_{cabin,air}$ = paramètre de température de l'air de l'habitacle du véhicule,
$T_{body\_surf,i}$ = température de la surface du corps du passager mesurée,
et
$z_i$ ; $z_1$ à $z_6$ = coefficients.

9. Système d'évaluation (10) selon l'une quelconque des revendications précédentes, le dispositif d'évaluation de paramètres (12) étant configuré pour évaluer un paramètre de fréquence respiratoire (RR) sur la base de fluctuations de température mesurées dans la zone sub-nasale du visage d'un passager de véhicule et/ou sur la base de fluctuations de température provoquées par un flux d'air alternatif à travers le nez du passager provoqué par une activité respiratoire, et/ou le dispositif d'évaluation de paramètres (12) étant configuré pour évaluer un paramètre de fréquence respiratoire (RR) en utilisant un filtre passe-bande et/ou une transformation de Fourier rapide.

10. Système d'évaluation (10) selon l'une quelconque des revendications précédentes, le dispositif d'évaluation de paramètres (12) étant configuré pour évaluer un paramètre de taux métabolique (MR) sur la base d'un rythme cardiaque évalué et/ou d'un paramètre de rythme cardiaque (HR), et/ou le dispositif d'évaluation de paramètres (12) étant configuré pour évaluer un rythme cardiaque (HR) et/ou un paramètre de rythme cardiaque (HR) sur la base de mesures et/ou de résultats de surveillance du front du passager de véhicule, de préférence par la mesure des fluctuations de température provoquées par les variations du flux sanguin à travers les vaisseaux sanguins hypodermiques et/ou par des mesures avec une fréquence d'images d'au moins 2 images par seconde, plus préférablement d'au moins 3 images par seconde, et/ou par des mesures des variations de couleur de la peau du front d'un passager de véhicule, de préférence agrandies par des algorithmes d'agrandissement vidéo eulériens, et/ou le dispositif d'évaluation de paramètres étant configuré pour évaluer un paramètre de taux métabolique (MR) en utilisant un filtre passe-bande et/ou une transformation de Fourier rapide.

11. Système d'évaluation (10) selon l'une quelconque des revendications précédentes, le dispositif d'évaluation de paramètres (12) étant configuré pour évaluer un paramètre d'état d'humeur (MS) sur la base d'un suivi optique de repères faciaux, de préférence les yeux, les sourcils, le nez et/ou la bouche d'un passager de véhicule, et/ou sur la base d'une transformation de positions géométriques relatives de repères faciaux suivis.

12. Système d'évaluation (10) selon l'une quelconque des revendications précédentes 1 à 11, comprenant un dispositif capteur d'état (36), le dispositif capteur d'état (36) comprenant :

- au moins une unité de capteur optique (38) pour détecter la longueur d'onde dans le spectre visible, et
- au moins une unité de capteur de température (40) pour détecter des longueurs d'onde dans le spectre infrarouge à ondes longues,
- la densité de pixels de l'unité de capteur optique (38) étant supérieure à la densité de pixels de l'unité de capteur de température (40).

**13.** Système d'évaluation (10) selon la revendication 12, la densité minimale de pixels de l'unité de capteur de température (40) étant définie par la fonction :

$$\frac{pixel}{deg}\Big|min = d\,\frac{\pi}{180°}\,\frac{HP}{WF}$$

avec :

d = distance entre le capteur de température et un visage de passager,
HP = nombre minimal de pixels horizontaux pour la résolution de visage, et
WF = largeur moyenne du visage d'un passager.

**14.** Procédé d'évaluation de l'état d'un passager de véhicule avec un système d'évaluation (10) selon l'une quelconque des revendications 1 à 13, le procédé comprenant les étapes de :

- évaluation d'une pluralité de paramètres d'un ensemble de paramètres de l'intérieur du véhicule et/ou du passager de véhicule, l'ensemble de paramètres comprenant :

- un paramètre de température de l'air de l'habitacle du véhicule ($T_{cabin,air}$),
- un paramètre de température de surface de l'habitacle du véhicule ($T_{cabin,rad}$),
- un paramètre d'état corporel thermique du passager (TBS),
- un paramètre de taux métabolique du passager (MR),
- un paramètre de niveau d'habillement du passager (CL),
- un paramètre de fréquence respiratoire du passager (RR), et
- un paramètre d'état d'humeur du passager (MS) ; et

- évaluation d'une valeur d'état de passager (CV) sur la base de la pluralité de paramètres évalués ;
- l'évaluation de la valeur d'état (CV) étant effectuée en appliquant un facteur de pondération pour un paramètre évalué ; le facteur de pondération étant compris dans l'une des plages suivantes :

$$- 10 < a < 10,$$

$$- 10 < b < 10,$$

$$0 < c < 8,$$

$$0 < d < 8,$$

$$0 < e < 5,$$

$$- 1 < f < 0,$$

et

$$0,2 < g < 3 ;$$

avec :

a = facteur de pondération du paramètre de température de l'air de l'habitacle du véhicule,
b = facteur de pondération du paramètre de température de surface de l'habitacle du véhicule,

c = facteur de pondération du paramètre d'état corporel thermique du passager,
d = facteur de pondération du paramètre de taux métabolique du passager,
e = facteur de pondération du paramètre de niveau d'habillement du passager,
f = facteur de pondération du paramètre de fréquence respiratoire du passager, et
g = facteur de pondération du paramètre d'état d'humeur du passager

**caractérisé en ce qu'**une valeur d'état de passager (CV) est évaluée sur la base de la fonction :

$$CV = a \cdot (T_{cabin,air} - T_{cabin,air,ref}) + b \cdot (T_{cabin,rad} - T_{cabin,rad,ref}) + c \cdot TBS + d \cdot (MR - MR_{ref}) + e \cdot (CL - CL_{ref}) + f \cdot (RR - RR_{ref}) + g \cdot MS$$

avec :

CV = valeur d'état de passager,
$T_{cabin,air,ref}$ = paramètre de température de l'air de l'habitacle du véhicule de référence,
$T_{cabin,rad,ref}$ = paramètre de température de surface de l'habitacle du véhicule de référence,
$MR_{ref}$ = valeur de référence du taux métabolique du passager,
$CL_{ref}$ = valeur de référence du niveau d'habillement du passager,
$RR_{ref}$ = valeur de référence de la fréquence respiratoire du passager.

Fig. 1

**Fig. 2**

36

38 40

50

54

52 58

FoV

56

48

Fig. 3

**Fig. 4a**

**Fig. 4b**

**Fig. 4c**

**REFERENCES CITED IN THE DESCRIPTION**

**Patent documents cited in the description**

- US 5674007 A